# EUROPÄISCHE PATENTSCHRIFT

(11) **EP 1 404 691 B1**
(45) Veröffentlichungstag und Bekanntmachung des Hinweises auf die Patenterteilung: **09.03.2005**
(21) Anmeldenummer: 02743207.9
(22) Anmeldetag: 14.06.2002
(51) Int. Cl.: C07H 13/04

(54) **VERFAHREN ZUR HERSTELLUNG VON PERACYLIERTEN 1-O-GLYCOSIDEN**
METHOD FOR PRODUCING PERACYLATED 1-O-GLYCOSIDES
PROCEDE DE PRODUCTION DE 1-O-GLYCOSIDES PERACYLES

(30) Priorität: 11.07.2001 DE 10135098
(43) Veröffentlichungstag der Anmeldung: 07.04.2004
(73) Patentinhaber: SCHERING AKTIENGESELLSCHAFT, 13342 Berlin (DE)
(72) Erfinder: PLATZEK, Johannes, 12621 Berlin (DE); GRASKE, Klaus-Dieter, 12169 Berlin (DE); NIEDBALLA, Ulrich, 14195 Berlin (DE)
(86) Internationale Anmeldenummer: PCT/EP2002/006609
(87) Internationale Veröffentlichungsnummer: WO 2003/006474

(56) Entgegenhaltungen:
- EP-A- 0 882 733
- WO-A-01/68659
- WO-A-88/00592
- SUGAWARA T ET AL: "SYNTHESIS OF OMEGA-(METHOXYCARBONYL)ALKYL AND 9-(METHOXYCARBONYL)-3,6-DIOXANONYL GLYCOPYRANOSIDES FOR THE PREPARATION OF CARBOHYDRATE-PROTEIN CONJUGATES" CARBOHYDRATE RESEARCH, ELSEVIER SCIENTIFIC PUBLISHING COMPANY. AMSTERDAM, NL, Bd. 230, Nr. 1, 1992, Seiten 117-150, XP001018683 ISSN: 0008-6215 in der Anmeldung erwähnt
- SCHMIDT R R ET AL: "1-O-ALKYLATION OF D-GLUCOPYRANOSE" JOURNAL OF CARBOHYDRATE CHEMISTRY, NEW YORK, NY, US, Bd. 3, Nr. 1, 1984, Seiten 67-84, XP001021740 ISSN: 0732-8303
- DATABASE WPI Section Ch, Week 199443 Derwent Publications Ltd., London, GB; Class B03, AN 1994-347142 XP002226505 & JP 06 271597 A (DDS KENKYUSHO KK), 27. September 1994 (1994-09-27)
- LOCKHOFF OSWALD: "An access to glycoconjugate libraries through multicomponent reactions" ANGEWANDTE CHEMIE. INTERNATIONAL EDITION, VERLAG CHEMIE. WEINHEIM, DE, Bd. 37, Nr. 24, 1998, Seiten 3436-3439, XP002171115 ISSN: 0570-0833

## Beschreibung

Die Erfindung betrifft ein neues Verfahren zur Herstellung von peracylierten 1-O-Glycosiden der allgemeinen Formel I, das in den Patentansprüchen näher gekennzeichnet ist. Das erfindungsgemäße Verfahren geht von kostengünstigen Ausgangsmaterialien aus, liefert gute Ausbeuten und erlaubt die Herstellung von peracylierten Sacchariden mit 1-O-funktionalisierten Seitenketten im großen Maßstab.

Peracylierte Saccharid-Derivate sind wertvolle Zwischenprodukte in der Synthesechemie. Vor allem die pharmazeutische Chemie verwendet derartige Bausteine sehr häufig, da viele hochpotente und selektive Pharmaka Zuckerreste tragen. So sind beispielsweise im Journal of Drug Targeting 1995, Vol. 3, pp. 111-127 Anwendungen des sogenannten "Glycotargetings" beschrieben. Sogenannte "multi-antennary sugar chains" sind in Chemistry Letters 1998, S. 823 beschrieben. Durch Clustering von Zuckereinheiten wird die Carbohydrat-Rezeptor-Wechselwirkung bei der Zell-Zell-Interaktion wesentlich verbessert. Die Synthese von Galaktosiden mit hoher Affinität zum Asialoglycoprotein Receptor ist im J. Med. Chem. 1995, 38, p. 1538 publiziert worden (siehe auch Int. J. Peptide Protein Res. 43, 1994, p. 477). Hier werden derivatisierte Galactosen mit funktionalisierten Seitenketten hergestellt, die anschließend an verschiedene andere Moleküle gehängt werden können. Eine gute Übersicht über die Verwendung von Sacchariden als Basis der Glycobiologie ist in Acc. Chem. Res. 1995, 321 gegeben worden . Auch für die Synthese von LewisX Mimetika (Tet. Lett. Vol. 31, 5503) dienen funktionalisierte Monosaccharide als Vorstufen (siehe auch JACS 1996, 118, 6826).

Die Verwendung von derivatisierten Monosacchariden als Zwischenstufen für potentielle Pharmazeutika ist in Current Medicinal Chemistry, 1995, 1, 392 gut dargestellt worden. Perbenzylierte-1-OH-Zucker-Derivate (Galactose, Glucose) werden auch in der Synthese von herzaktiven Glycosiden (Digitoxin-Konjugate) eingesetzt. Die 1-O-Glycosidierung erfolgt hier via Trichloracetimidat und BF₃-Katalyse (J. Med. Chem. 1986, 29, p. 1945). Zur Herstellung immobilisierter Zucker-Liganden (z. B. Verknüpfung an HSA) werden funktionalisierte, geschützte Monosaccharide eingesetzt (Chemical Society Reviews 1995, p. 413).

Ziel einer Gruppe von Synthesen ist es, über eine 1-O-Glycosidierungsreaktion zusätzlich Funktionalität ins Zuckermolekül einzuführen. Hier sind vor allem endständige COOH-, Amino- oder OH-Gruppen von Interesse, da diese in Folgeschritten weiter umgesetzt werden können.

Die Herstellung von 1-O-Glycosiden erfolgt in den meisten Fällen nach klassischen Methoden, wie z.B. nach der von Koenigs-Knorr, Helferich oder der von R.R. Schmidt beschriebenen Trichloracetimidat-Methode [W. Koenigs und E. Knorr, Ber. dtsch. chem. Ges. 34 (1901) 957; B. Helferich u. J. Goendeler, Ber. dtsch. Chem. Ges. 73, (1940) 532; B. Helferich, W. Fiel und F. Eckstein, Chem. Ber. 94 (1961), 491; B. Helferich u. W.M. Müller, Chem. Ber. 1970, 103, 3350; G. Wulff, G. Röhle und W. Krüger, Ang. Chem. Internat. Edn., 1970, 9, 455; J. M. Berry u. G.G.S. Duthon, Canad. J. Chem. 1972, 50, 1424; R. R. Schmidt, Angew. Chem. 1986, 98, 213.] .

Allen diesen Methoden ist gemeinsam, daß die 1-Hydroxylgruppe in eine reaktive Form überführt wird, die letztlich als Abgangsgruppe dient. Unter Lewissäure-Katalyse (teilweise in stöchiometrischer Menge), erfolgt die eigentliche Umsetzung mit einem Alkohol zum 1-O-Glycosid. Für derartige Umsetzungen gibt es zahlreiche Beispiele in der Literatur.

So ist bei der Herstellung des Immunostimulans KRN-7000 (Kirin Brewery) die Kondensation von Tetra-O-benzyl-β-D-galactopyranosyl-bromid mit einem primären Alkohol, dessen Hydroxylgruppe am Ende einer di-Hydroxy-Amido-C-Kette sitzt (in DMF/Toluol unter Lewissäure Katalyse) ein zentraler Schritt (Drug of the Future 1997, 22(2), p. 185). In dem japanischen Patent JP 95-51764 ist die Umsetzung von 1-O-Acetyl-2,3,4-tri-O-benzyl-L-fucopyranose mit Polyoxyethylen-30-Phytosterol (BPS-30, NIKKO Chem., Japan) unter Trimethylsilylbromid/Zinktriflat-Katalyse beschrieben worden. In Bull. Chem. Soc. 1982, 55(4), p. 1092-6 sind 1-O-Glycosidierungen von Perbenzyl-Zuckern unter Titantetrachlorid-Katalyse in Dichlormethan beschrieben.

In Liebigs Ann. Org. Bioorg. Chem.; EN; 9; 1995; 1673-1680 ist die Herstellung von 3,4,5-Trisbenzyloxy-2-benzyloxymethyl-6-(2-hexadecyloxyethoxy)-tetrahydropyran beschrieben. Ausgehend von 2,3,4,6-Tetra-O-benzyl-D-glucopyranose wird die 1-O-Glycosidierung unter Verwendung von Bu₄NBr, CoBr₂, Me₃SiBr und Molekularsieb in Methylenchlorid innerhalb von 60 Stunden durchgeführt .

Ein Tetrabenzylderivat, das eine endständige, als Methylester geschützte Carboxylgruppe enthält, ist in Carbohydr. Res.; EN; 230; 1; 1992; 117 beschrieben. Die Carboxylgruppe kann danach freigesetzt und weiter umgesetzt werden. Zur Glycosidierung wird Silbercarbonat in Dichlormethan verwendet. Die Verwendung des teuren Silbercarbonats limitiert die Ansatzgröße und macht ein wirtschaftliches up-scaling fast unmöglich. Das gleiche Problem gilt für die nachfolgende Verbindung, die in Tetrahedron Lett. 30, 44, 1989, p. 6019 beschrieben worden ist. Hier wird 2,3,4,6-Tetra-O-benzyl-D-mannosyl-bromid in Nitromethan mit 2-Benzyloxyethanol unter Zurhilfenahme von Quecksilbercyanid zum 1-O-Glycosid umgesetzt. Die Verwendung von Quecksilbercyanid in pilot-plant-Anlagen ist problematisch und aus umweltpolitischer Sicht abzulehnen.

Die in neuester Zeit beschriebenen Substanzbibliotheken für das Hochdurchsatz-Screening verwenden sehr häufig Saccharide (Angew. Chemie 1995, 107, 2912). Hier ist es das Ziel, Zuckerbausteine in geschützter Form vorliegen zu haben, die eine funktionelle Gruppe, wie z.B. -COOH, oder -NH₂ tragen, welche z.B. in einer automatisierten Synthese umgesetzt werden können. Die Bausteine , die dafür Verwendung finden, sind von Lockhoff, Angew. Chem. 1998, 110(24), S. 3634 beschrieben worden. Vor allem die 1-O-Essigsäure von Perbenzyl-Glucose ist hier von Bedeutung. Die Herstellung erfolgt über 2 Stufen, via Trichloracetimidat und Umsetzung mit Hydroxyessigsäureethylester, BF₃-Katalyse in THF und anschließender Verseifung mit NaOH in MeOH/THF. Die Gesamtausbeute über 2 Stufen beträgt allerdings nur 59 %.

In der gleichen Publikation wird auch die Herstellung eines 1-O-(Aminoethyl)-Glycosids der perbenzylierten Glucose beschrieben. Die Umsetzung erfolgt, wieder ausgehend vom Trichloracetimidat, durch Umsetzung mit N-Formylaminoethanol unter BF₃-Katalyse in THF und anschließender Verseifung in MeOH/THF. Die Gesamtausbeute ist auch hier relativ gering, sie beträgt 45 %.

Ein 1-O-(Aminoethyl)-Derivat der Perbenzylxylose wird in Carbohydrate Research 1997, 298, p. 173 als Zwischenprodukt durchlaufen. Die Synthese ist jedoch sehr langwierig, da sie vom 1-Brom-peracetat der Xylose startet. Die eigentlliche 1-O-Glycosidierung erfolgt über einen 1-Phenylthioether, der mit 2-Azidoethanol unter DMTST-Katalyse (= Dimethyl (methyltio)-sulfonium-triflat) in Dichlormethan umgesetzt wird (Gesamtstufenzahl: 7). Die Gesamtausbeute ist mit unter 40 % für eine industrielle Anwendung nicht geeignet.

Im Übersichtsartikel von R.R. Schmidt in Angew. Chem. 1986, 98, S. 213-236 werden direkte Umsetzungen von 1-OH-Perbenzylglucose und -ribose mit 2-Halogenestern und Triflaten beschrieben. Als Base wird Natriumhydrid in THF oder Benzol verwendet (Chem. Ber. 1982, 115), die Ausbeuten liegen zwischen 40 und 55 %. Auch der Einsatz von Natriumhydrid in Dioxan oder Kalium-tert.-butylat in THF (beides bei Raumtemperatur) ist zur 1-O-Alkylierung mit Triflaten beschrieben (Angew. Chem. 1986, 98, S. 218). Die strengstens einzuhaltenden wasserfreien Reaktionsbedingungen stellen eine große Hürde beim up-scaling derartiger Alkylierungen dar.

Alle bisher bekannten Verfahren haben den großen Nachteil, daß sich ein up-scaling des Prozesses nicht ohne weiteres bewerkstelligen läßt. Die Verwendung von Lewis-Säuren bei der 1-O-Glycosidierung sowie von Natriumhydrid bei der 1-O-Alkylierung erfordert stets wasserfreie Reaktionsbedingungen, was bei großen Ansätzen immer mit Schwierigkeiten verbunden ist. Auch die Aufarbeitung und Entsorgung der Reaktionshilfsstoffe (Hg/Cyanid/etc.) ist in vielen Fällen ein Problem.

Aufgabe der Erfindung war es deshalb, ein Verfahren bereitzustellen, mit dem peracylierte Saccharide mit 1-O-funktionalisierter Seitenkette in größerem Maßstab, preiswert und umweltfreundlich hergestellt werden können.

Die Aufgabe der Erfindung wird gemäß dem in den Ansprüchen angegebenen Verfahren gelöst, mit welchem peracylierte 1-O-Glycoside der allgemeinen Formel I 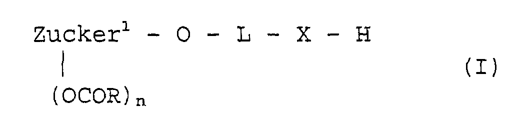 hergestellt werden können. Gemäß Definition der Erfindung bedeutet Zucker¹ in der allgemeinen Formel I ein in 1-OH-Position funktionalisiertes Monosaccharid, wobei es sich hierbei auch um Desoxyzucker handeln kann, die anstelle einer oder mehrerer OH-Gruppen ein H-Atom enthalten. In einer bevorzugten Ausführungsform der Erfindung bedeutet der Zucker in der allgemeinen Formel I ein Monosaccharid mit 5 oder 6 C-Atomen, z. B. Glucose, Mannose, Galactose, Ribose, Arabinose oder Xylose oder deren Desoxyzucker wie beispielsweise 6-Desoxygalactose (Fucose) oder 6-Desoxy-Mannose (Rhamnose).

Der Rest -COR stellt die Acylgruppe dar, die in Abhängigkeit vom eingesetzten Monosaccharid oder dessen Desoxyform mindestens zweifach vorhanden ist und beim Einsatz von Di-, Tri- oder Polysacchariden entsprechend mehrfach vorhanden ist. Als Reste R kommen aliphatische und aromatische Gruppen in Betracht wie beispielsweise Methyl, Ethyl, Isopropyl, tButyl oder Phenyl.
Der Rest X bedeutet -COO- oder -NH-. Im Ergebnis des erfindungsgemäßen Verfahrens werden also Alkohole, Carbonsäuren oder Amine der allgemeinen Formel I erhalten.

Der Rest L kann eine geradkettige, verzweigte, gesättigte oder ungesättigte C₁-C₃₀-Kohlenstoffkette bedeuten, die gegebenenfalls unterbrochen ist durch 1-10 Sauerstoffatome, 1-3 Schwefelatome, 1-2 Phenylen-, 1-2 Phenylenoxy-, 1-2 Phenylendioxygruppen, einen Thiophen-, Pyrimidin- oder Pyridinrest und/oder gegebenenfalls substituiert ist mit 1-3 Phenyl-, 1-3 Carboxyl-, 1-5 Hydroxy-, 1-5 O-C₁-C₇-alkyl-, 1-3 Aminogruppen, 1-3 CF₃-Gruppen oder 1-10 Fluoratomen. Im Sinne der Erfindung bevorzugte Reste L sind 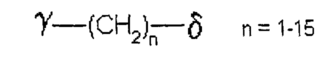 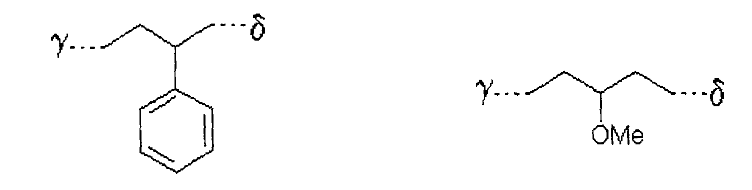 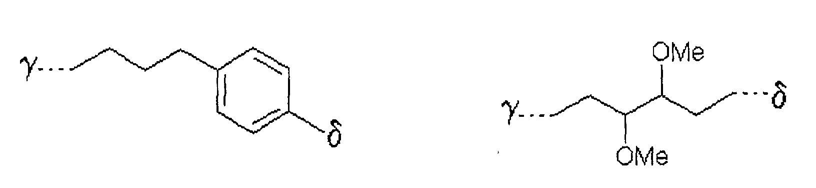 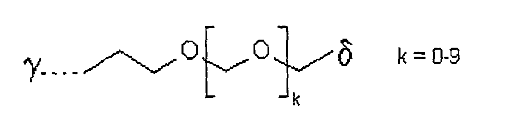 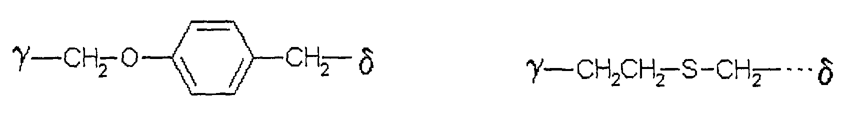 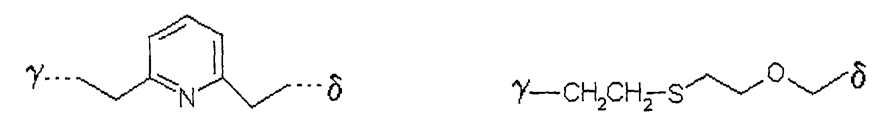 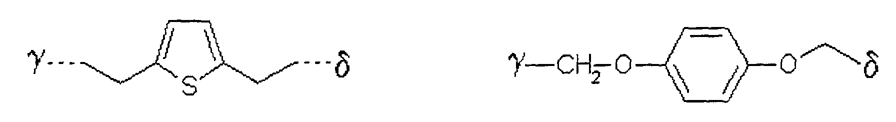 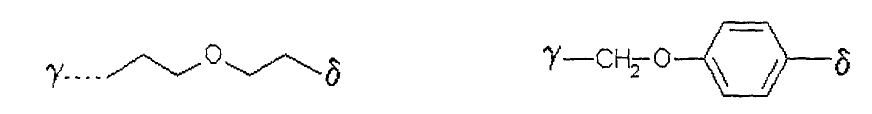 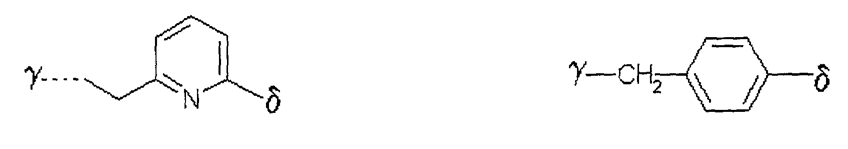 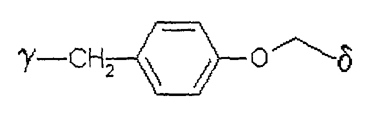 wobei γ die Verknüpfungsstelle am Zucker bedeutet und δ die Verknüpfungsstelle zum Rest X ist. Ein besonders bevorzugter Linker L ist die -CH₂-Gruppe.

Zur Herstellung der peracylierten 1-O-Glycoside der allgemeinen Formel I wird ein peracylierter 1-OH-Zucker der allgemeinen Formel II 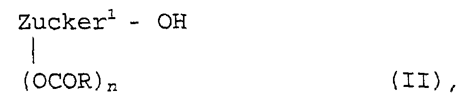 worin Zucker, R und n die oben angegebene Bedeutung haben, in einem organischen Lösungsmittel gelöst und mit einem Alkylierungsreagenz der allgemeinen Formel III

Nu - L - X - Sg (III),

worin Nu ein Nucleofug bedeutet, L und X die genannte Bedeutung haben und Sg eine Schutzgruppe ist, in Gegenwart einer Base und gegebenenfalls eines Phasentransfer-Katalysators umgesetzt. Als Nucleofug können im Alkylierungsreagenz der allgemeinen Formel III beispielsweise die Reste - Cl, -Br, -J, -OTs, -OMs, -OSO₂CF₃, -OSO₂C₄F₉ oder -OSO₂C₈F₁₇ enthalten sein.

Bei der Schutzgruppe Sg handelt es sich um eine übliche Säure- oder Amin-Schutzgruppe, je nachdem ob X den Rest -COO- oder - NH- bedeutet. Diese Schutzgruppen sind dem Fachmann gut vertraut (Protective Groups in Organic Syntheses, second Edition, T.W.Greene and P.G.M. Wuts, John Wiley & Sons Inc., New York 1991) .

Die erfindungsgemäße Umsetzung kann bei Temperaturen von 0-50°C, vorzugsweise von 0°C bis Raumtemperatur erfolgen. Die Reaktionszeiten betragen von 10 Minuten bis 24 Stunden, vorzugsweise von 20 Minuten bis 12 Stunden.

Die Base wird entweder in fester Form, vorzugsweise fein gepulvert oder flüssig zugesetzt. Als bevorzugte Basen dienen Cäsiumcarbonat, Kaliumcarbonat, 1,8-Diazabicyclo-[5.4.0]undec-7-en (DBU), 1,5-Diazabicyclo- [2.2.2]octan (DBN), 1,4-Diazabicyclo[2.2.2]octan (DABCO), Kalium-t-butoxid und Natrium-t-butoxid, Natriumcarbonat oder ein Gemisch aus Cäsiumcarbonat und Kaliumcarbonat oder Natriumcarbonat.

Als organische Lösungsmittel können im erfindungsgemäßen Alkylierungsverfahren beispielsweise Acetonitril, Dioxan, Tetrahydrofuran, Diethoxymethan, 1,2-Dimethoxyethan, Diethylenglycoldimethylether, Benzol, Formamid, Hexan, Toluol, Dimethylformamid, Dimethylacetamid, Cyclohexan, CF3-Benzol, Diethylether, Dichlormethan, Methyl-t-butylether (MTB), Dimethylsulfoxid, Sulfolan oder deren Gemische eingesetzt werden.

Als Phasentransfer-Katalysatoren dienen im erfindungsgemäßen Verfahren die für diesen Zweck bekannten quartären Ammonium- oder Phosphoniumsalze oder auch Kronenether wie z. B. [15]-Krone-5- oder [18]-Krone-6. Vorzugsweise kommen quartäre Ammoniumsalze mit vier gleichen oder verschiedenen Kohlenwasserstoffgruppen am Kation, ausgewählt aus Methyl, Ethyl, Propyl, Isopropyl, Butyl oder Isobutyl in Frage. Die Kohlenwasserstoffgruppen am Kation müssen groß genug sein, um eine gute Löslichkeit des Alkylierungsreagenzes im organischen Lösungsmittel zu gewährleisten. Erfindungsgemäß besonders bevorzugt wird N(Butyl)₄⁺-Cl⁻, N(Butyl)₄⁺-HSO₄⁻, aber auch N(Methyl)₄⁺-Cl⁻ eingesetzt.

Nach erfolgter Umsetzung kann die Aufarbeitung des Reaktionsgemisches durch Isolierung des noch geschützten Endproduktes und nachfolgende übliche Abspaltung der Schutzgruppe zum Endprodukt der allgemeinen Formel I erfolgen. Bevorzugt ist es jedoch, das noch geschützte Endprodukt nicht zu isolieren, sondern das Lösungsmittel zu entfernen, den Rückstand in einem neuen, für die Abspaltung der Schutzgruppe geeigneten Lösungsmittel aufzunehmen und hier die Abspaltung durchzuführen. Die Verfahrensweise zur Abspaltung der Schutzgruppe und zur Regenerierung der Säure-, Amino- oder Hydroxygruppe ist dem Fachmann gut bekannt.

Handelt es sich z. B. bei der Schutzgruppe Sg um eine Säureschutzgruppe, die das acide Proton der Carboxygruppe blockiert, also z. B. um Methyl, Ethyl, Benzyl oder um tert-Butyl, so wird die Säure üblicherweise durch alkalische Hydrolyse regeneriert. In dem Verfahren der Erfindung sind jedoch die alkoholischen Hydroxylgruppen ebenfalls als Ester geschützt. Als Schutzgruppe für die Carbonsäure bleibt neben Allyl- und Silyl- die Benzylgruppe verfügbar. Diese Schutzgruppe läßt sich bequem durch katalytische Hydrierung entfernen .Als Katalysator hat sich dabei Palladium (10%) auf Aktivkohle bewährt.

Als Hydroxyschutzgruppen (in L) kommen z.B. Benzyl-, 4-Methoxybenzyl-, 4-Nitrobenzyl-, Trityl-, Diphenylmethyl-, Trimethylsilyl-, Dimethyl-tert-butylsilyl oder Diphenyl-tert-butylsilylgruppen in Frage.
Die Hydroxygruppen können auch z.B. als THP-Ether, α-Alkoxyethylether, MEM-Ether oder als Ester mit aromatischen oder aliphatischen Carbonsäuren, wie z.B. Essigsäure oder Benzoesäure, vorliegen. Im Fall von Polyolen können die Hydroxygruppen auch in Form von Ketalen mit z. B. Aceton, Acetaldehyd, Cyclohexanon oder Benzaldehyd geschützt sein.
Die Hydroxyschutzgruppen können nach den dem Fachmann bekannten Literaturmethoden, z. B. durch Hydrogenolyse, Säurebehandlung der Ether und Ketale, Alkalibehandlung der Ester oder Behandlung der Silylschutgruppen mit Fluorid freigesetzt werden (siehe z. B. Protective Groups in Organic Syntheses, secon Edition, T.W. Greene and P.G.M. Wuts, John Wiley & Sons, Inc., New Yourk, 1991).

Die NH₂-Gruppen lassen sich in vielfältiger Weise schützen und wieder freilegen. Das N-Trifluoracetylderivat wird durch Kalium- oder Natriumcarbonat in Wasser [H. Newman, J. Org. Chem., 30:287 (1965), M. A. Schwartz et al., J. Am. Chem. Soc., 95 G12 (1973)] oder einfach durch Ammoniaklösung gespalten [M. Imazama u. F. Eckstein, J. Org. Chem., 44:2039 (1979)]. Ebenfalls milde zu spalten ist das tert-Butyloxycarbonylderivat: es genügt Rühren mit Trifluoressigsäure [B. F. Lundt et al., J. Org. Chem., 43:2285 (1978)]. Sehr groß ist die Gruppe der hydrogenolytisch oder reduzierend zu spaltenden NH₂-Schutzgruppen: Die N-Benzylgruppe ist bequem mit Wasserstoff/Pd-C zu spalten [W.H. Hartung und R. Simonoff, Org. Reactions VII, 263 (1953)], was auch für die Tritylgruppe [L. Zervas, et al., J. Am. Chem. Soc., 78:1359 (1956)] und die Benzyloxycarbonylgruppe gilt [M.Bergmann u. L. Zervas Ber. 65:1192 (1932)].
Von den Silylderivaten werden die leicht spaltbaren tert-Butyldiphenylsilylverbindungen [L. E. Overman et al., Tetrahedron Lett., 27:4391 (1986)], wie auch die 2-(Trimethylsilyl)-ethyl carbamate [L. Grehn et al., Angew. Chem. Int. Ed. Engl., 23:296 (1983)] und die 2-Trimethylsilylethansulfonamide [R.S. Garigipati u. S.M. Weinreb, J. Org. Chem., 53:4134 (1988)] verwendet, die mit Fluoridionen gespalten werden können. Besonders leicht spaltbar ist das 9-Fluorenylmethyl-carbamat: Die Spaltung erfolgt mit Aminen wie Piperidin, Morpholin, 4-Dimethylaminopyridin, aber auch mit Tetrabutylammoniumfluorid [L. A. Corpino et al., J. Org. Chem., 55:1673 (1990); M. Ueki u. M. Amemiya, Tetrahedron Lett., 28:6617(1987)].

Die Isolierung des erhaltenen Endproduktes der allgemeinen Formel I (Amin oder Carbonsäure) erfolgt ebenfalls nach üblichen und dem Fachmann gut bekannten Methoden.

So wird beispielsweise im Fall der Säureschutzgruppe das Lösungsmittel aus der Hydrolysereaktion abgedampft und der Rückstand in einem aprotischen Lösungsmittel aufgenommen. Durch Ansäuern mit einer wäßrigen Säurelösung wird der pH auf ca. 2-4 eingestellt und danach die organische Phase abgetrennt. Mittels Kristallisation oder Chromatographie kann nun das peracylierte 1-O-Glycosid gewonnen werden.

Gewünschtenfalls können die erhaltenen Verbindungen der allgemeinen Formel I auch in üblicher Weise in ihre Salze überführt werden.

Die Ausbeuten der Verbindungen der allgemeinen Formel I, die mit dem erfindungsgemäßen Verfahren erzielt werden können, sind gut. Sie liegen für bekannte Verbindungen, bei denen ein Vergleich mit dem Stand der Technik möglich ist, über den Ausbeuten des Standes der Technik. So erhält man beispielsweise (WO 96/35700) bei der Umsetzung von Acetobromglucose mit Glycolsäuremethylester unter dem Einfluss von Quecksilberoxid und Quecksilber (II) bromid nach Koenigs-Knorr die 1-O-Methyloxycarbonylmethyl-2,3,4,6-tetra-O-acetylglucopyranose in 60% Ausbeute. Die Verseifung zur freien Säure wäre mit einem weiteren Ausbeuteverlust verbunden. In EP 882733 wird ebenfalls die Darstellung dieser Verbindung beschrieben, allerdings ohne Ausbeuteangabe. Nach dem erfindungsgemäßen Verfahren wird die Säure auch in einem zweistufigen Verfahren erhalten. Hier beträgt die Ausbeute jedoch 78 % ( Beispiel 24, diese Anmeldung).

Die entsprechende Benzyloxycarbonylmethyl-2,3,4,6-0-tetraacetyl-galactopyranose wird in JP 6-271597 beschrieben.

Neben den hohen Ausbeuten bietet das erfindungsgemäße Verfahren auch den Vorteil, daß es von kostengünstigen Startmaterialien ausgeht, ein scale-up des Prozesses ermöglicht und eine leichte Isolierung der Endprodukte erlaubt.

Die Ausgangsmaterialien sind Kaufware oder aus käuflichen Vorstufen leicht erhältlich. So ist die Tetra-2,3,4,6-O-acetyl D-glucopyranose bequem aus der Pentaacetylverbindung durch partielle Hydrolyse mit Benzylamin erhältlich (Organikum, 4. Auflage, VEB Deutscher Verlag der Wissenschaften Berlin 1964, S.376). Bei Fluka sind Methyl-D-manno-pyranosid und Methyl-D-galactopyranosid Katalogware. Durch Acylierung und Spaltung des Glycosids sind 2,3,4,6-Tetra-O-acyl-D-mannose bzw. - galactose erhältlich.
Über die Sequenz-Methylglycosid-Peracyl-methylglycosid-Peracyl-1-OH-saccharid lassen sich die Peracyl-1-OH-Derivate der Pentosen (Ribose, Arabinose), Hexosen und Desoxyhexosen (Rhamnose, Fucose) gewinnen.

Die erfindungsgemäß hergestellten Verbindungen sind wertvolle Zwischenprodukte in der Synthesechemie. So können sie beispielsweise zum Aufbau von Kohlenhydratdendrimeren, zur Synthese von NMR-Kontrastmitteln und zur Einführung von Zuckerresten in Pharmaka Verwendung finden.

Das erfindungsgemäße Verfahren soll nachfolgend an Ausführungsbeispielen näher erläutert werden.

### Beispiel 1

### 2,3,4,6-Tetra-O-acetyl-1-O-carboxymethyl-mannopyranose

Eine Mischung aus 34,83 g (100 mmol) 2,3,4,6-Tetra-O-acetylmannopyranose, 1,70 g (5 mmol) Tetrabutylammoniumhydrogensulfat und 82,93g (600 mmol) feingepulvertes Kaliumcarbonat in 350 ml Dioxan werden auf 0°C abgekühlt. Bei 0°C tropft man 34,36 g (150 mmol) Bromessigsäurebenzylester über 10 Minuten ,unter starkem Rühren zu. Man rührt eine Stunde bei 0°C. Man gibt 250 ml MTB (Methyl-tert.butylether) zu, filtriert vom Feststoff ab und dampft das Filtrat im Vakuum zur Trockne ein. Der Rückstand wird in 500 ml Ethanol aufgenommen. Man gibt 5 g Palladium (10 %) auf Aktivkohle dazu und hydriert bis die Lösung keinen Wasserstoff mehr aufnimmt. Man filtriert vom Katalysator ab, wäscht mit Ethanol nach und engt die vereinigten Lösungen im Vakuum zu Trockene ein.° Der Rückstand wird an Kieselgel chromatographiert (Laufmittel: Dichlormethan/nHexan/ Ethanol/Essigsäure = 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
34,5 g (85 % d. Th., über 2 Stufen) eines farblosen, zähen Öls

| Elementaranalyse: | | |
|---|---|---|
| ber. | C 47,29 | H 5,46 |
| gef. | C 47,38 | H 5,55 |

### Beispiel 2

### 2,3,4,6-Tetra-O-benzoyl-1-O-carboxymethyl-mannopyranose

Eine Mischung aus 59,66 g (100 mmol) 2,3,4,6-Tetra-O-benzoylmannopyranose, 1,7 g (5 mmol) Tetrabutylammoniumhydrogensulfat und 63,59 g (600 mmol) feingepulvertes Natriumcarbonat in 350 ml 1,2-Dimethoxyethan werden auf 0°C abgekühlt. Bei 0°C tropft man 34,36 g (150 mmol) Bromessigsäurebenzylester über 10 Minuten unter starkem Rühren zu. Man rührt eine Stunde bei 0°C. Man gibt 250 ml MTB (Methyl-tert.Butylether) zu, filtriert vom Feststoff ab und dampft das Filtrat im Vakuum zur Trockne ein. Der Rückstand wird in 500 ml Ethanolgelöst. Man gibt 5 g Palladium (10 %) auf Aktivkohle dazu und hydriert bis zur Beendung der Wasserstoffaufnahme. Dann filtriert man vom Katalysator ab, wäscht mit Ethanol nach und engt die vereinigten Lösungen im Vakuum zur Trockne ein. Der Rückstand wird an Kieselgel chromatographiert (Laufmittel: Dichlormethan/n=Hexan/ Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
53,03 g (81 % d. Th., über 2 Stufen) eines farblosen, zähen Öls

| Elementaranalyse: | | |
|---|---|---|
| ber. | C 66,05 | H 4,62 |
| gef. | C 66,19 | H 4,78 |

### Beispiel 3

### 2,3,4,6-Tetra-O-pivaloyl -1-O-(5-carboxypentyl)-mannopyranose

Eine Mischung aus 51,66 g (100 mmol) 2,3,4,6-Tetra-O-pivaloyl -mannopyranose, 0,55 g (5 mmol) Tetramethylammoniumchlorid und 82,93 g (600 mmol) feingepulvertes Kaliumcarbonat in 350 ml Diethylenglycoldimethylether wird auf 10°C abgekühlt. Bei 10°C tropft man 35,7 g (150 mmol) 6-Bromhexansäurebenzylester innerhalb von 10 Minuten unter starkem Rühren zu. Man rührt 2 Stunden bei 10°C. Man gibt 250 ml MTB (Methyl-tert.Butylether) zu, filtriert vom Feststoff ab und dampft das Filtrat im Vakuum zur Trockne ein. Der Rückstand wird in 500 ml Ethanol aufgenommen, mit 5 g Palladium (10%) auf Aktivkohle versetzt und bis zur Beendigung der Wasserstoffaufnahme hydriert. Man filtriert vom Katalysator ab, wäscht mit Ethanol nach und engt die vereinigten Lösungen im Vakuum zur Trockene ein. Der Rückstand wird an Kieselgel chromatographiert (Laufmittel: Dichlormethan/n= Hexan/ Ethanol/ Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
49,83 g (79 % d. Th., über 2 Stufen) eines farblosen Feststoffs

| Elementaranalyse: | | |
|---|---|---|
| ber. | C 60,93 | H 8,63 |
| gef | C 61,12 | H 8,78 |

### Beispiel 4

### 2,3,4,6-Tetra-O-acetyl-1-O-(1-phenyl-1-carboxy-eth-2-yl)-manopyranose

Eine Mischung aus 34,83 g (100 mmol) 2,3,4,6-Tetra-O-acetylmannopyranose, 1,39 g (5 mmol) Tetrabutylammoniumchlorid und 35,8 g (110 mmol) feingepulvertes Cäsiumcarbonat in 400 ml Dioxan werden auf 0°C abgekühlt. Bei 0°C tropft man 47,88 g (150 mmol) 2-Phenyl-3-brompropionsäurebenzylester, gelöst in 30 ml Dioxan über 10 Minuten unter starkem Rühren zu. Man rührt eine Stunde bei 0°C. Man gibt 250 ml MTB (Methyl-tert.Butylether) zu, filtriert vom Feststoff ab und dampft das Filtrat im Vakuum zur Trockne ein. Der Rückstand wird in 500 ml Tetrahydrofuran aufgenommen. Man gibt 5 g Palladium (10%) auf Aktivkohle dazu und hydriert bis zur Beendigung der Wasserstoffaufnahme. Dann filtriert man vom Katalysator ab, wäscht mit wenig Tetrahydrofuran nach, vereinigt die organischen Lösungen und engt sie im Vakuum zur Trockene ein. Der Rückstand wird an Kieselgel chromatographiert (Laufmittel: Dichlormethan/n= Hexan/ Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
39,22 g (79 % d. Th., über 2 Stufen) eines farblosen Feststoffs

| Elementaranalyse: | | |
|---|---|---|
| ber. | C 55,64 | H 5,68 |
| gef. | C 55,81 | H 5,84 |

### Beispiel 5

### 2,3,4,6-Tetra-O-benzoyl-1-O-carboxymethyl-mannopyranose

Eine Mischung aus 59,66 g (100 mmol) 2,3,4,6-Tetra-O-benzoylmannopyranose, 1,39 g (5 mmol) Tetrabutylammoniumchlorid und 15,2 g (110 mmol) wasserfreiem Kaliumcarbonat in 500 ml Dimethylformamid wird 0°C abgekühlt. Bei 0°C tropft man 36,92 g (200 mmol Chloressigsäurebenzylester über 20 Minuten unter starkem Rühren zu. Man rührt eine Stunde bei 10°C. Man gibt 250 ml Methyl-tert.Butylether zu, trennt die organische Phase ab filtriert sie und engt sie im Vakuum zur Trockene ein..Der Rückstand wird in 500 ml Ethanol aufgenommen und mit 5 g Palladium (10 %) auf Aktivkohle versetzt. Man hydriert bis zur Beendigung der Wasserstoffaufnahme. Man filtriert vom Katalysator ab, wäscht mit wenig Ethanol nach und engt die vereinigten Lösungen im Vakuum zur Trockene ein. Der Rückstand wird an an Kieselgel chromatographiert (Laufmittel: Dichlormethan/n= Hexan/Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
53,82 g (82 % d. Th., über 2 Stufen) eines farblosen, zähen Öls

| Elementaranalyse: | | |
|---|---|---|
| ber. | C 66,05 | H 4,62 |
| gef. | C 66,21 | H 4,73 |

### Beispiel 6

### 2,3,4,6-Tetra-O-acetyl-1-O-(4-carboxybutyl)-glucopyranose

Eine Mischung aus 34,83 g (100 mmol) 2,3,4,6-Tetra-O-acetylglucopyranose 1,39 g (5 mmol) Tetrabutylammoniumchlorid und 15,22 g (100 mmol) DBU in 300 ml Tetrahydrofuran wird auf 0°C abgekühlt. Bei 0°C tropft man 54,37 g (150 mmol) 5-Tosyloxypentancarbonsäurebenzylester, gelöst in 40 ml Tetrahydrofuran über 30 Minuten unter starkem Rühren zu. Man rührt 3 Stunden bei 0°C. Man gibt 300 ml MTB (Methyl-tert.Butylether) zu, filtriert vom Feststoff ab und dampft das Filtrat im Vakuum zur Trockne ein. Der Rückstand wird in 500 ml Tetrahydrofuran aufgenommen. Man gibt 5 g Palladium (10 %) auf Aktivkohle dazu und hydriert bis zur Beendigung der Wasserstoffaufnahme. Dann filtriert man vom Katalysator ab, wäscht mit wenig Tetrahydrofuran nach und engt im Vakuum zur Trockene ein. Der Rückstand wird an Kieselgel chromatographiert (Laufmittel: Dichlormethan/n= Hexan/ Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
34,98 g (78 % d. Th., über 2 Stufen) eines farblosen Feststoffs

| Elementaranalyse: | | |
|---|---|---|
| ber. | C 50,89 | H 6,29 |
| gef. | C 51,02 | H 6,41 |

### Beispiel 7

### 2,3,4,6-Tetra-O-pivaloyl-1-O-carboxymethyl-glucopyranose

Eine Mischung aus 54,1 g (100 mmol) 2,3,4,6-Tetra-O-pivaloyl-glucopyranose, 1,39 g (5 mmol) Tetrabutylammoniumchlorid und 11,22 g Kalium t butoxid (100 mmol) in 500 ml Dimethylacetamid wird auf 0°C abgekühlt. Bei 0°C tropft man 34,36 g (150 mmol Bromessigsäurebenzylester über 20 Minuten unter starkem Rühren zu. Man rührt 0,5 Stunden bei 0°C. Man gibt 250 Toluol zu, trennt die organische Phase ab, filtriert sie. und engt sie im Vakuum zur Trockene ein. Der Rückstand wird in 400 ml Methanol aufgenommen, mit 5 g Palladium (10%) auf Aktivkohle versetzt und bis zur Beendigung der Wasserstoffaufnahme hydriert.Man filtriert vom Katalysator ab, wäscht mit wenig Methanol nach und engt die vereinigten organischen Lösungen im Vakuum zur Trockene ein. Der Rückstand wird an Kieselgel chromatographiert (Laufmittel: Dichlormethan/n-Hexan/ Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
47,12 g (82 % d. Th., über 2 Stufen) eines farblosen, zähen Öls

| Elementaranalyse: | | |
|---|---|---|
| ber. | C 58,52 | H 8,07 |
| gef. | C 58,69 | H 8,19 |

### Beispiel 8

### 2,3,4,6-Tetra-O-acetyl-1-O-(10-carboxydecyl)-glucopyranose

Eine Mischung aus 34,83 g (100 mmol) 2,3,4,6-Tetra-Oacetylglucopyranose, 0,55 g (5 mmol) Tetramethylammoniumchlorid und 12,42 g (100 mmol) DBN in 350 ml Benzol wird auf 0°C abgekühlt. Bei 0°C tropft man 53,30 g (150 mmol) 11-Bromundecansäurebenzylester, gelöst in 50 ml Benzol über 30 Minuten unter starkem Rühren zu. Man rührt 2 Stunden bei 20°C. Man gibt 250 ml Methyl-tert.Butylether zu, filtriert vom Feststoff ab und dampft das Filtrat im Vakuum zur Trockne ein. Der Rückstand wird in 500 ml Ethanol aufgenommen. Man gibt 5 g Palladium (10 %) auf Aktivkohle dazu und hydriert bis zur Beendigung der Wasserstoffaufnahme. Man filtriert vom Katalysator ab, wäscht mit wenig Ethanol nach und engt die vereinigten Lösungen im Vakuum zur Trockene ein Der Rückstand wird an Kieselgel chromatographiert (Laufmittel: Dichlormethan/n-Hexan/ Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
41,54 g (78 % d. Th., über 2 Stufen) eines farblosen Feststoffs

| Elementaranalyse: | | |
|---|---|---|
| ber. | C 56,38 | H 7,57 |
| gef. | C 56,42 | H 7,81 |

### Beispiel 9

### 2,3,4,6-Tetra-O-acetyl-1-O-carboxymethyl-galactopyranose

Eine Mischung aus 34,83 g (100 mmol) 2,3,4,6-Tetra-O-acetylmannopyranose, 1,39 g (5 mmol) Tetrabutylammoniumchlorid und 9,62 g (100 mmol) Natrium t butoxid in 350 ml Dimethylsulfoxid wird auf 0°C abgekühlt. Bei 0°C tropft man 36,92 g (200 mmol Chloressigsäurebenzylester über 20 Minuten unter starkem Rühren zu. Man rührt eine Stunde bei 10°C. Man gibt 250 ml Methyl-tert.Butylether zu, trennt die organische Phase ab und filtriert sie. Das Lösungsmittel wird im Vakuum abdestilliert. Der Rückstand wird in 500 ml Ethanol aufgenommen. Man gibt 5 g Palladium (10%) auf Aktivkohle dazu und hydriert bis zur Beendigung der Wasserstoffaufnahme. Man filtriert vom Katalysator ab, wäscht mit wenig Ethanol nach und engt im Vakuum zur Trockene ein. Der Rückstand wird an Kieselgel chromatographiert (Laufmittel: Dichlormethan/n= Hexan/Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
33,32 g (82 % d. Th., über 2 Stufen) eines farblosen, zähen Öls

| Elementaranalyse: | | |
|---|---|---|
| ber. | C 47,29 | H 5,46 |
| gef. | C 47,38 | H 5,57 |

### Beispiel 10

### 2,3,4,6-Tetra-O-acetyl-1-O-[1-(4-carboxy)-phenyl-prop-3-yl-galactopyranose

Eine Mischung aus 34,83 g (100 mmol) 2,3,4,6-Tetra-O-acetylgalactopyranose, 1,39 g (5 mmol) Tetrabutylammoniumchlorid und 67,31 g (600 mmol) 1,4-Diazabicyclo[2.2.2]octan in 300 ml Acetonitril wird auf 10°C abgekühlt. Bei 10°C tropft man 52,26 g (150 mmol) 4-(3-Methansulfonyloxy-propyl)-benzoesäurebenzylester, gelöst in 50 ml Tetrahydrofuran über 30 Minuten unter starkem Rühren zu. Man rührt 2 Stunden bei 10°C. Man gibt 300 ml MTB (Methyl-tert.Butylether) zu, filtriert vom Feststoff ab und dampft das Filtrat im Vakuum zur Trockne ein. Der Rückstand wird in 500 ml. Tetrahydrofuran gelöst, und man gibt 5 g Palladium (10%) auf Aktivkohle dazu und hydriert bis zur Beendigung der Wasserstoffaufnahme. Dann filtriert man vom Katalysator ab, wäscht mit wenig Tetrahydrofuran nach und engt die vereinigten Lösungen im Vakuum zur Trockene ein. Der Rückstand wird an Kieselgel chromatographiert (Laufmittel: Dichlormethan/n=Hexan/ Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
39,31 g (77 % d. Th., über 2 Stufen) eines farblosen Feststoffs

| Elementaranalyse: | | |
|---|---|---|
| ber. | C 56,47 | H 5,92 |
| gef. | C 56,55 | H 6,04 |

### Beispiel 11

### 2,3,5-Tri-O-benzoyl-1-O-carboxymethyl-ribofuranose

Eine Mischung aus 46,25 g (100 mmol) 2,3,5-Tri-O-benzoylribofuranose, 1,39 g (5 mmol) Tetrabutylammoniumchlorid in 400 ml Methyl-tert.butylether und 82,93 g (600 mmol) Kaliumcarbonat wird auf 0°C abgekühlt. Bei 0°C tropft man 22,91 g (150 mmol 2-Bromessigsäurebenzylester über 20 Minuten unter starkem Rühren zu. Man rührt eine Stunde bei 0°C. Man gibt 500 ml Methyl-tert.butylether dazu, trennt die organische Phase ab und extrahiert die wässrige Phase 2 mal mit 200 ml Methyl-tert.butylether. Das Lösungsmittel der vereinigten organischen Phasen wird über Natriumsulfat getrocknet, vom Trock- nungsmittel abfiltriert und im Vakuum abdestilliert. Der Rückstand wird in 500 ml Ethanol aufgenommen. Man gibt 5g Palladium (10%) auf Aktivkohle dazu und hydriert bis zur Beendigung der Wasserstoffaufnahme. Dann filtriert man vom Katalysator ab, wäscht mit wenig Ethanol nach und engt im Vakuum zur Trockene ein. Der Rückstand wird an Kieselgel chromatographiert (Laufmittel: Dichlormethan/n Hexan/Ethanol/Essigsäure= 20:5:3:0,5). Die produktenthaltenden Fraktionen werden eingedampft, in 200 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
42,68 g (82 % d. Th., über 2 Stufen) eines farblosen, zähen Öls

| Elementaranalyse: | | |
|---|---|---|
| ber. | C 64,61 | H 4,65 |
| gef. | C 64,72 | H 4,71 |

### Beispiel 12

### 2,3,5-Tri-O-benzoyl-1-O-(1-amino-eth-2-yl)-ribofuranose, Hydrochlorid

Eine Mischung aus 42,1 g (100 mmol) 2,3,5-Tri-O-benzoylribofuranose, 3,40 g (10 mmol) Tetrabutylammoniumhydrogensulfat und 63,94 g (600 mmol) feingepulvertes Natriumcarbonat in 350 ml Dioxan wird auf 10°C abgekühlt. Bei 10°C tropft man 45,63 g (150 mmol) N-(2-Bromethyl)-dibenzylamin, gelöst in 100 ml Benzol über 40 Minuten unter starkem Rühren zu. Man rührt 3 Stunden bei 10°C, gibt dann 300 ml Benzol zu, filtriert vom Feststoff ab und dampft das Filtrat im Vakuum zur Trockne ein. Der Filtratrückstand wird in 500 ml Ethanol gelöst, mit 5 g Pearlman-Katalysator (20 % Palladium auf Aktivkohle) versetzt und bis zur Beendigung der Wasserstoffaufnahme hydriert. Man filtriert vom Katalysator ab, wäscht mit wenig Ethanol nach und engt im Vakuum zur Trockene ein. Der Rückstand wird an Kieselgel chromatographiert (Laufmittel: Dichlormethan/Ethanol/Triethylamin= 20:2:0,1).
Die produkthaltigen Fraktionen werden vereinigt und im Vakuum zur Trockene eingeengt. Man nimmt in absolutem Diethylether auf und fällt das Produkt mit etherischer Salzsäure. als Hydrochlorid.
Ausbeute:
42,27 g (78 % d. Th., über 2 Stufen) eines farblosen Feststoffs

| Elementaranalyse: | | | | |
|---|---|---|---|---|
| ber. | C 62,05 | H 5,21 | N 2,58 | Cl 6,54 |
| gef. | C 62,19 | H 5,28 | N 2,66 | Cl 6,47 |

### Beispiel 13

### 2,3,4,6-Tetra-O-benzoyl-1-O-(1-amino-prop-3-yl)-galactopyranose, Hydrochlorid

Eine Mischung aus 42,1 g (100 mmol) 2,3,4,6-Tetra-O-benzoylgalactopyranose, 1,7 g (5 mmol) Tetrabutylammoniumhydrogensulfat und 82,93 g (600 mmol) feingepulvertes Kaliumcarbonat in 350 ml 1,2-Dimethoxyethan werden auf 10°C abgekühlt. Bei 10°C tropft man 47,74 g (150 mmol) N-(3-Brompropyl)-dibenzylamin, gelöst in 100 ml 1,2-Dimethoxyethan über 40 Minuten unter starkem Rühren zu. Man rührt 3 Stunden bei 10°C. Man gibt 300 ml Benzol zu, filtriert vom Feststoff ab und dampft das Filtrat im Vakuum zur Trockne ein. Der Rückstand wird in 500 ml Ethanol gelöst, mit 5 g Pearlman-Katalysator versetzt und bis zur Beendigung der Wasserstoffaufnahme hydriert. Man saugt vom Katalysator ab, wäscht mit wenig Ethanol nach und engt im Vakuum zur Trockene ein Der Rückstand wird an Kieselgel chromatographiert (Laufmittel: Dichlormethan/Ethanol/Triethylamin= 20:2:0,1).
Die produkthaltigen Fraktionen werden vereinigt und im Vakuum zur Trockene eingeengt. Man nimmt den Rückstand in absolutem Diethylether auf und fällt das Produkt durch Zusatz von etherischer Salzsäure als Hydrochlorid.
Ausbeute:
53,14 g (77 % d. Th. über 2 Stufen) eines farblosen Feststoffs

| Elementaranalyse: | | | | |
|---|---|---|---|---|
| ber. | C 64,39 | H 5,26 | Cl 5,14 | N 2,03 |
| gef. | C 64,24 | H 5,34 | Cl 5,21 | N 2,11 |

### Beispiel 14

### 2,3,4,6-Tetra-O-acetyl-1-O-(1-amino-hex-6-yl)-mannopyranose, Hydrochlorid

Eine Mischung aus 34,83 g (100 mmol) 2,3,4,6-Tetra-O-acetylmannopyranose, 1,39 g (5 mmol) Tetrabutylammoniumchlorid und 35,84 g (110 mmol) Cäsiumcarbonat in 500 ml Dioxan werden auf 0°C abgekühlt. Bei 0°C tropft man 60,3 g (150 mmol 6-Bromhexylamin-N-(9-fluorenylmethoxy-carbonyl) über 30 Minuten unter starkem Rühren zu. Man rührt eine Stunde bei 0°C. Man gibt 300 ml Dichlormethan zu, trennt die organische Phase ab und filtriert sie. Man engt im Vakuum zur Trockene ein und nimmt den Rückstand in 500 ml Ethanol auf, gibt 5 g Palladium (10 %) auf Aktivkohle dazu und hydriert bis zur Beendigung der Wasserstoffaufnahme. Dann filtriert man vom Katalysator ab, wäscht mit wenig Ethanol nach und engt im Vakuum zur Trockene ein. Der Rückstand wird an Kieselgel chromatographiert (Laufmittel: Dichlormethan/ Ethanol/ Triethylamin= 20:2:0,1).
Die produkthaltigen Fraktionen werden vereinigt und im Vakuum zur Trockene eingeengt. Der Rückstand wird in absolutem Diethylether aufgenommen und mit etherischer Salzsäure versetzt. Das Produkt wird als Hydrochlorid erhalten.
Ausbeute:
38,23 g (79 % d. Th. über 2 Stufen) eines farblosen Feststoffs

| Elementaranalyse: | | | | |
|---|---|---|---|---|
| ber. | C 49,64 | H 7,08 | N 2,89 | Cl 7,33 |
| gef. | C 49,69 | H 7,20 | N 2,94 | Cl 7,48 |

### Beispiel 15

### 2,3,4-Tri-O- -1-O-(1-amino-but-4-yl)-fucopyranose, Hydrochlorid benzoyl

Eine Mischung aus 43,5 g (100 mmol) 2,3,4-Tri-O-benzoyl-6-desoxy-galactopyranose, 1,7 g (5 mmol) Tetrabutyl-ammoniumhydrogensulfat und 35,84 g (110 mmol) Cäsiumcarbonat in 400 ml Dichlormethan wird auf 0°C abgekühlt. Bei 10°C tropft man 47,4 g (150 mmol) 2-(Trimethylsilyl)-ethylsulfonsäure-N-(4-brombutyl)-amid, gelöst in 100 ml Dichlormethan über 30 Minuten unter starkem Rühren zu. Man rührt 2 Stunden bei 10°C. Man gibt 600 ml Dichlormethan zu, trennt die organische Phase ab, extrahiert die wässrige Phase 2 mal mit 200 ml Dichlormethan. Die vereinigten organischen Phasen werden über Natriumsulfat getrocknet. Man filtriert vom Trocknungsmittel und und destilliert das Lösungsmittel im Vakuum ab. Der Rückstand wird in 350 ml Acetonitril aufgenommen und 52,3 g (200 mmol) Tetrabutylammoniumflorid als Monohydrat zugegeben. Man rührt 3 Stunden bei 50 °C. Die Lösung wird zur Trockne eingeengt und der Rückstand an Kieselgel chromatographiert (Laufmittel: Dichlormethan/ Ethanol/ Triethylamin= 20:2:0,1).
Die produkthaltigen Fraktionen werden vereinigt und im Vakuum zur Trockene eingeengt. Der Rückstand wird in absolutem Diethylether gelöst und mit etherischer Salzsäure versetzt.
Das Produkt wird als Hydrochlorid erhalten.
Ausbeute:
45,56 g (78 % d. Th. über 2 Stufen) eines farblosen Feststoffs

| Elementaranalyse: | | | | |
|---|---|---|---|---|
| ber. | C 63,75 | H 5,87 | N 2,40 | Cl 6.07 |
| gef. | C 63,63 | H 5,91 | N 2,45 | Cl 6,18 |

### Beispiel 16

### 2,3,4,6-Tetra-O-pivaloyl-1-O-(3,6,9,12,15-pentaoxa-1-carboxy-hexadec-16-yl)-glucopyranose

Eine Mischung aus 51,66 g (100 mmol) 2,3,4,6-Tetra-O-pivaloylglucopyranose, 1,39 g (5 mmol) Tetrabutylammoniumchlorid und 63,59 g (600 mmol) feingepulvertes Natriumcarbonat in 350 ml Dioxan wird auf 0°C abgekühlt. Bei 0°C tropft man 91,37 g (130 mmol) 17-Tosyloxy-3,6,9,12,15-pentaoxaheptadecansäure-benzylester, gelöst in 100 ml Tetrahydrofuran über 50 Minuten unter starkem Rühren zu. Man rührt 3 Stunden bei 0°C. Man gibt 300 ml Dichlormethan zu, filtriert vom Feststoff ab und dampft das Filtrat im Vakuum zur Trockne ein. Der Rückstand wird in 500 ml Ethanol aufgenommen. Man gibt 5g Palladium (10 &) auf Aktivkohle dazu und hydriert bis zur Beendigung der Wasserstoffaufnahme. Dann saugt man vom Katalysator ab, wäscht mit wenig Ethanol nach und engt im Vakuum zur Trockene ein.
Der Rückstand wird an Kieselgel chromatographiert (Laufmittel: Dichlormethan/n Hexan/Ethanol/Essigsäure= 20:8:5:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
61,21 g (77 % d. Th., über 2 Stufen) eines farblosen Öls

| Elementaranalyse: | | |
|---|---|---|
| ber. | C 57,42 | H 8,37 |
| gef. | C 57,56 | H 8,29 |

### Beispiel 17

### 2,3,4,6-Tetra-O-acetyl-1-0-(1-hydroxy-eth-2-yl)-mannopyranose

Eine Mischung aus 34,83 g (100 mmol) 2,3,4,6-Tetra-O-acetylmannopyranose, 1,7 g (5 mmol) Tetrabutylammoniumhydrogensulfat und 82,93 g (600 mmol) feingepulvertes Kaliumcarbonat in 350 ml Diethoxymethan wird auf 0°C abgekühlt. Bei 0°C tropft man 32,26 g (150 mmol) 2-Benzyloxy-ethylbromid über 30 Minuten unter starkem Rühren zu. Man rührt 2 Stunden bei 0°C. Man gibt 300 ml Benzol zu, filtriert vom Feststoff ab und dampft das Filtrat im Vakuum zur Trockne ein. Der Rückstand wird in 500 ml Ethanol aufgenommen. Man gibt 5 g Palladium (10%) auf Aktivkohle dazu und hydriert bis zur Beendigung der Wasserstoffaufnahme. Dann saugt man vom Katalysatorab, wäscht mit wenig Ethanol nach und engt im Vakuum zur Trockene ein Der Rückstand wird an Kieselgel chromatographiert (Laufmittel: Dichlormethan/nHexan/ Ethanol= 20:8:2). Die produktenthaltenden Fraktionen werden eingedampft.
Ausbeute:
30,60 g (78 % d. Th., über 2 Stufen) eines farblosen, zähen Öls

| Elementaranalyse: | | |
|---|---|---|
| ber. | C 48,98 | H 6,17 |
| gef. | C 48,84 | H 6,03 |

### Beispiel 18

### 2,3,4,6-Tetra-O-pivaloyl -1-O-carboxymethyl-mannopyranose

Eine Mischung aus 51,66 g (100 mmol) 2,3,4,6-Tetra-O-pivaloyl-mannopyranose, 0,55 g (5 mmol) Tetramethylammoniumchlorid und 82,93 g (600 mmol) feingepulvertes Kaliumcarbonat in 350 ml Diethylenglycoldimethylether wird auf 10°C abgekühlt. Bei 10°C tropft man 36,65 g (160 mmol) 2-Bromessigsäurebenzylester innerhalb von 10 Minuten unter starkem Rühren zu. Man rührt 2 Stunden bei 10°C. Man gibt 250 ml MTB (Methyl-tert.Butylether) zu, filtriert vom Feststoff ab und dampft das Filtrat im Vakuum zur Trockne ein. Der Rückstand wird in 500 ml Ethanol aufgenommen, mit 5 g Palladium (10%) auf Aktivkohle versetzt und bis zur Beendigung der Wasserstoffaufnahme hydriert. Man filtriert vom Katalysator ab, wäscht mit Ethanol nach und engt die vereinigten Lösungen im Vakuum zur Trockene ein. Der Rückstand wird an Kieselgel chromatographiert (Laufmittel: Dichlormethan/n= Hexan/ Ethanol/ Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
45,40 g (79 % d. Th., über 2 Stufen) eines farblosen Feststoffs

| Elementaranalyse: | | |
|---|---|---|
| ber. | C 58,52 | H 8,07 |
| gef | C 58,44 | H 8,18 |

### Beispiel 19

### 2,3,4,6-Tetra-O-acetyl-1-O-(2-phenyl-1-carboxy-eth-2-yl)-manopyranose

Eine Mischung aus 34,83 g (100 mmol) 2,3,4,6-Tetra-O-acetylmannopyranose, 1,39 g (5 mmol) Tetrabutylammoniumchlorid und 35,8 g (110 mmol) feingepulvertes Cäsiumcarbonat in 400 ml Diethylether wird auf 0°C abgekühlt. Bei 0°C tropft man 47,88 g (150 mmol) 2-Phenyl-3-brompropionsäurebenzylester, gelöst in 30 ml Diethylether über 10 Minuten unter starkem Rühren zu. Man rührt eine Stunde bei 0°C. Man gibt 250 ml MTB (Methyl-tert.Butylether) zu, filtriert vom Feststoff ab und dampft das Filtrat im Vakuum zur Trockne ein. Der Rückstand wird in 500 ml Tetrahydrofuran aufgenommen. Man gibt 5 g Palladium (10%) auf Aktivkohle dazu und hydriert bis zur Beendigung der Wasserstoffaufnahme. Dann filtriert man vom Katalysator ab, wäscht mit wenig Tetrahydrofuran nach, vereinigt die organischen Lösungen und engt sie im Vakuum zur Trockene ein. getrocknet, das Lösungsmittel im Vakuum abdestilliert Der Rückstand wird an Kieselgel chromatographiert (Laufmittel: Dichlormethan/n= Hexan/ Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
39,22 g (79 % d. Th., über 2 Stufen) eines farblosen Feststoffs

| Elementaranalyse: | | |
|---|---|---|
| ber. | C 55,64 | H 5,68 |
| gef. | C 55,81 | H 5,84 |

### Beispiel 20

### 2,3,4,6-Tetra-O-benzoyl-1-O-carboxymethyl-mannopyranose

Eine Mischung aus 59,66 g (100 mmol) 2,3,4,6-Tetra-O-benzoylmannopyranose, 1,39 g (5 mmol) Tetrabutylammoniumchlorid und 15,2 g (110 mmol) wasserfreiem Kaliumcarbonat in 500 ml Diethoxymethan wird 0°C abgekühlt. Bei 0°C tropft man 36,92 g (200 mmol 2-Chloressigsäurebenzylester über 20 Minuten unter starkem Rühren zu. Man rührt eine Stunde bei 10°C. Man gibt 250 ml Methyl-tert.Butylether zu, trennt die organische Phase ab filtriert sie und engt sie im Vakuum zur Trockene ein..Der Rückstand wird in 500 ml Ethanol aufgenommen und mit 5 g Palladium (10 %) auf Aktivkohle versetzt. Man hydriert bis zur Beendigung der Wasserstoffaufnahme. Man filtriert vom Katalysator ab, wäscht mit wenig Ethanol nach und engt die vereinigten Lösungen im Vakuum zur Trockene ein. Der Rückstand wird an an Kieselgel chromatographiert (Laufmittel: Dichlormethan/n= Hexan/Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
53,82 g (82 % d. Th., über 2 Stufen) eines farblosen, zähen Öls

| Elementaranalyse: | | |
|---|---|---|
| ber. | C 66,05 | H 4,62 |
| gef. | C 66,21 | H 4,73 |

### Beispiel 21

### 2,3,4,6-Tetra-O-acetyl-1-O-carboxymethyl-glucopyranose

Eine Mischung aus 34,83 g (100 mmol) 2,3,4,6-Tetra-O-acetylglucopyranose 1,39 g (5 mmol) Tetrabutylammoniumchlorid und 15,22 g (100 mmol) DBU in 300 ml Tetrahydrofuran wird auf 0°C abgekühlt. Bei 0°C tropft man 34,36 g (150 mmol) 2-Bromessigsäurebenzylester, gelöst in 40 ml Tetrahydrofuran über 30 Minuten unter starkem Rühren zu. Man rührt 3 Stunden bei 0°C. Man gibt 300 ml MTB (Methyl-tert.Butylether) zu, filtriert vom Feststoff ab und dampft das Filtrat im Vakuum zur Trockne ein. Der Rückstand wird in 500 ml Tetrahydrofuran aufgenommen. Man gibt 5 g Palladium (10 %) auf Aktivkohle dazu und hydriert bis zur Beendigung der Wasserstoffaufnahme. Dann filtriert man vom Katalysator ab, wäscht mit wenig und Tetrahydrofuran nach und engt im Vakuum zur Trockene ein. Der Rückstand wird an Kieselgel chromatographiert (Laufmittel: Dichlormethan/n= Hexan/ Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
31,69 g (78 % d. Th., über 2 Stufen) eines farblosen Feststoffs

| Elementaranalyse: | | |
|---|---|---|
| ber. | C 47,29 | H 5,46 |
| gef. | C 47,22 | H 5,61 |

### Beispiel 22

### 2,3,4,6-Tetra-O-pivaloyl-1-O-carboxymethyl-glucopyranose

Eine Mischung aus 54,1 g (100 mmol) 2,3,4,6-Tetra-O-pivaloylglucopyranose, 1,39 g (5 mmol) Tetrabutylammoniumchlorid und 11,22 g Kalium t butoxid (100 mmol) in 500 ml Dimethylacetamid ird auf 0°C abgekühlt. Bei 0°C tropft man 34,36 g (150 mmol 2-Bromessigsäurebenzylester über 20 Minuten unter starkem Rühren zu. Man rührt eine Stunde bei 0°C. Dann gibt man 250 ml Toluol dazu, filtriert vom Feststoffab, engt im Vakuum zur Trockene ein, nimmt den Rückstand in 500 ml Ethanol auf, versetzt mit 5 g Palladium (10 %) auf Aktivkohle und hydriert bis zur Beendigung der Wasserstoffaufnahme. Man filtriert vom Katalysator ab, wäscht mit wenig Ethanol nach und engt die vereinigten organischen Lösungen im Vakuum zur Trockene ein Der Rückstand wird an Kieselgel chromatographiert (Laufmittel: Dichlormethan/n-Hexan/ Ethanol/ Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
47,12 g (82 % d. Th., über 2 Stufen) eines farblosen, zähen Öls

| Elementaranalyse: | | |
|---|---|---|
| ber. | C 58,52 | H 8,07 |
| gef. | C 58,69 | H 8,19 |

### Beispiel 23

### 2,3,4,6-Tetra-O-acetyl-1-O-carboxymethyl-glucopyranose

Eine Mischung aus 34,83 g (100 mmol) 2,3,4,6-Tetra-O-acetylglucopyranose, 0,55 g (5 mmol) Tetramethylammoniumchlorid und 12,42 g (100 mmol) DBN in 350 ml Benzol werden auf 0°C abgekühlt. Bei 0°C tropft man 34,36 g (150 mmol) 2-Bromessigsäurebenzylester, gelöst in 50 ml Benzol über 30 Minuten unter starkem Rühren zu. Man rührt 2 Stunden bei 20°C. Man gibt 250 ml Methyl-tert.Butylether zu, filtriert vom Feststoff ab und dampft das Filtrat im Vakuum zur Trockne ein. Der Rückstand wird in 500 ml Ethanol aufgenommen. Man gibt 5 g Palladium (10 %) auf Aktivkohle dazu und hydriert bis zur Beendigung der Wasserstoffaufnahme. Man filtriert vom Katalysator ab, wäscht mit wenig Ethanol nach und engt die vereinigten Lösungen im Vakuum zur Trockene ein. Der Rückstand wird an Kieselgel chromatographiert (Laufmittel: Dichlormethan/n-Hexan/ Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
31,69 g (78 % d. Th., über 2 Stufen) eines farblosen Feststoffs

| Elementaranalyse: | | |
|---|---|---|
| ber. | C 47,29 | H 5,46 |
| gef. | C 47,42 | H 5.60 |

## Patentansprüche

1. Verfahren zur Herstellung von peracylierten 1-O-Glycosiden der allgemeinen Formel I 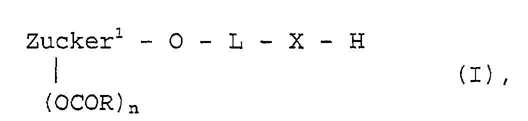 in der
Zucker¹ ein in 1-OH-Position funktionalisiertes Monosaccharid ist,
R Methyl, Ethyl, Propyl, Isopropyl, tButyl, Phenyl darstellt,
n 2, 3 oder 4 bedeutet,
X -COO- oder -NH- bedeutet
und
L eine geradkettige, verzweigte, gesättigte oder ungesättigte C₁-C₃₀-Kohlenstoffkette bedeutet, die gegebenenfalls unterbrochen ist durch 1-10 Sauerstoffatome, 1-3 Schwefelatome, 1-2 Phenylen-, 1-2 Phenylenoxy-, 1-2 Phenylendioxygruppen, einen Thiophen-, Pyrimidin- oder Pyridinrest und/oder gegebenenfalls substituiert ist mit 1-3 Phenyl-, 1-3 Carboxyl-, 1-5 Hydroxy-, 1-5 O-C₁-C₇-alkyl-, 1-3 Aminogruppen, 1-3 CF₃-Gruppen oder 1-10 Fluoratomen
oder deren Salzen
**dadurch gekennzeichnet, daß**
ein peracylierter 1-OH-Zucker der allgemeinen Formel II 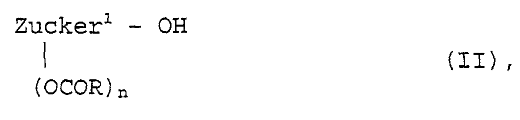 worin Zucker¹, R und n die angegebene Bedeutung haben,
mit einem Alkylierungsreagenz der allgemeine Formel (III)
Nu - L - X - Sg (III),
worin Nu ein Nucleofug bedeutet, L und X die genannte Bedeutung haben und Sg eine Schutzgruppe darstellt,
in einem organischen Lösungsmittel in Gegenwart einer Base und gegebenenfalls eines Phasentransfer-Katalysators bei einer Temperatur von 0-50°C umgesetzt wird, anschließend die Schutzgruppe abgespalten wird und das erhaltene Reaktionsprodukt gegebenenfalls in ein Salz überführt wird.

2. Verfahren nach Anspruch 1,
**dadurch gekennzeichnet, daß**
als peracylierter 1-OH-Zucker der allgemeinen Formel II ein peracyliertes Monosaccharid mit 5 bis 6 C-Atomen oder dessen Desoxy-Verbindung eingesetzt wird.

3. Verfahren nach Anspruch 1 oder 2,
**dadurch gekennzeichnet, daß**
als peracylierter 1-OH-Zucker der allgemeinen Formel II peracylierte Glucose, Mannose, Galactose, Ribose, Arabinose, Xylose, Fucose oder Rhamnose eingesetzt wird.

4. Verfahren nach einem der Ansprüche 1 bis 3,
**dadurch gekennzeichnet, daß**
als Alkylierungsreagenz der allgemeinen Formel III ein solches eingesetzt wird, in welchem das Nucleofug die Reste -Cl, -Br, -J, -OTs, -OMs, -OSO₂CF₃, -OSO₂C₄F₉ oder -OSO₂C₈F₁₇ bedeutet.

5. Verfahren nach einem der Ansprüche 1 bis 4,
**dadurch gekennzeichnet, daß**
als Alkylierungsreagenz der allgemeinen Formel III ein solches eingesetzt wird, in welchem der Rest L 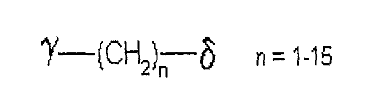 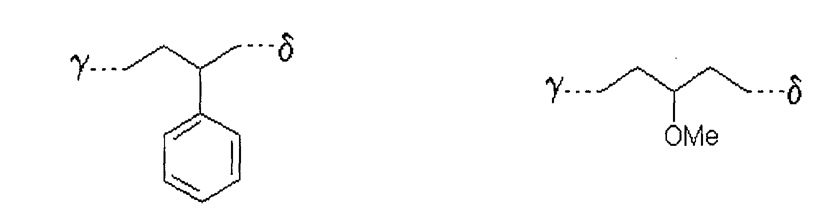 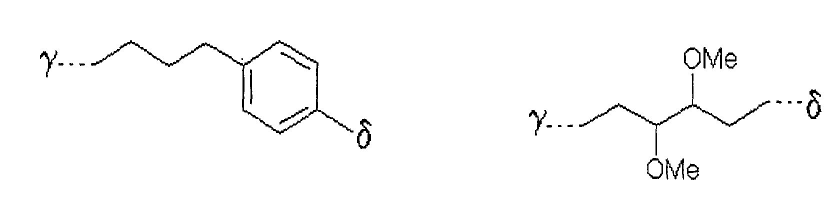 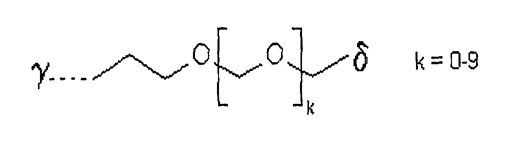 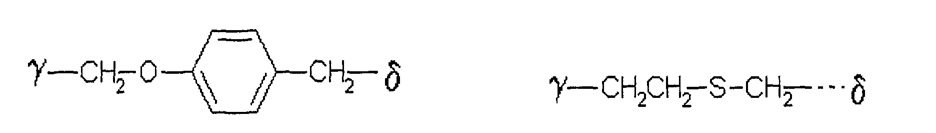 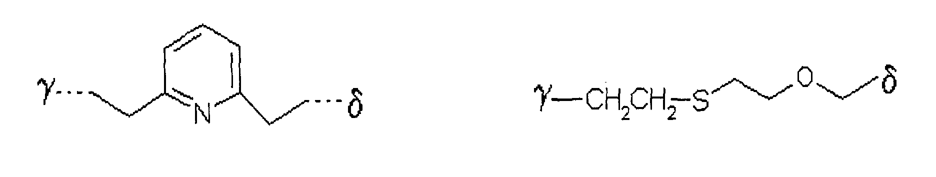 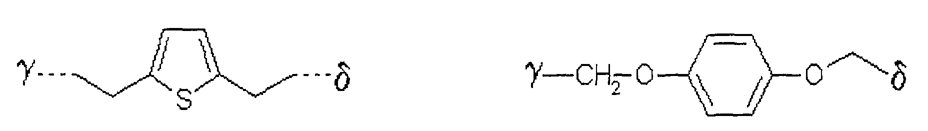 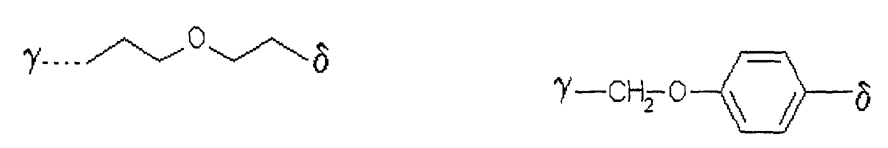 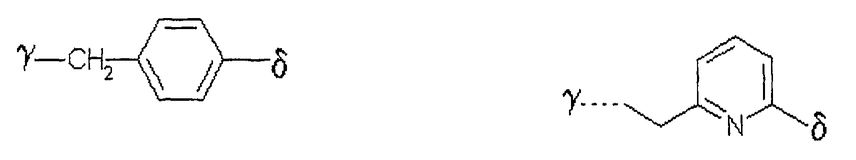 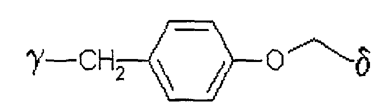 bedeutet,
wobei γ die Verknüpfungsstelle am Zucker bedeutet und δ die Verknüpfungsstelle zum Rest X ist.

6. Verfahren nach einem der Ansprüche 1 bis 5,
**dadurch gekennzeichnet,daß**
als organisches Lösungsmittel Dioxan,1,2-Dimethoxyethan, Diethylenglycoldimethylether, Diethoxymethan, Tetrahydrofuran, Acetonitril, Formamid, Dimethylformamid, Dimethylacetamid, Benzol, Toluol, CF3-Benzol, Hexan, Cyclohexan, Diethylether, Dichlormethan Methyl-t-butylether (MTB), Dimethylsulfoxid, Sulfolan oder deren Gemische eingesetzt wird.

7. Verfahren nach einem der Ansprüche 1 bis 6,
**dadurch gekennzeichnet, daß**
als Phasentransfer-Katalysator ein quartäres Ammonium- oder Phosphoniumsalz oder ein Kronenether eingesetzt wird, vorzugsweise ein quartäres Ammoniumsalz.

8. Verfahren nach einem der Ansprüche 1 bis 7, **dadurch**
**gekennzeichnet,daß**
als Base Kaliumcarbonat, Natriumcarbonat Cäsiumcarbonat, 1,8-Diazabi-cyclo[5.4.0]undec-7-en (DBU), 1,5-Diazabicyclo[4.3.0]non-5-en (DBN), 1,4-Diazabicyclo[2.2.2]-octan (DABCO), Kalium-t-butoxid, Natrium-t-butoxid oder ein Gemisch aus Cäsiumcarbonat und Kaliumcarbonat oder Natriumcarbonat eingesetzt wird.

9. Verfahren nach einem der Ansprüche 1 bis 7, **dadurch gekennzeichnet, daß**
die Base in fester oder flüssiger Form zugesetzt wird.

10. Verbindungen:
Carboxymethyl-2,3,4,6-Tetra-O-acetyl-mannopyranose
Carboxymethyl-2,3,4,6-Tetra-O-pivaloyl-mannopyranose
Carboxymethyl-2,3,4,6-Tetra-O-benzoyl-mannopyranose

## Claims

1. Process for the production of peracylated 1-O-glycosides of general formula I 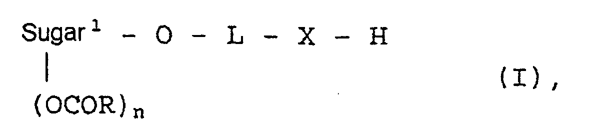 in which
sugar¹ is a monosaccharide that is functionalized in 1-OH position,
R represents methyl, ethyl, propyl, isopropyl, t-butyl, phenyl,
n means 2, 3 or 4,
X means -COO- or -NH-
and
L means a straight-chain, branched, saturated or unsaturated C ₁-C₃₀ carbon chain, which optionally is interrupted by 1-10 oxygen atoms, 1-3 sulphur atoms, 1-2 phenylene groups, 1-2 phenyleneoxy groups, 1-2 phenylenedioxy groups, a thiophene radical, pyrimidine radical or pyridine radical, and/or optionally is substituted with 1-3 phenyl groups, 1-3 carboxyl groups, 1-5 hydroxy groups, 1-5 O-C 1-C₇-alkyl groups, 1-3 amino groups, 1-3 CF ₃ groups or 1-10 fluorine atoms
or their salts,
**characterized in that**
a peracylated 1-OH-sugar of general formula II 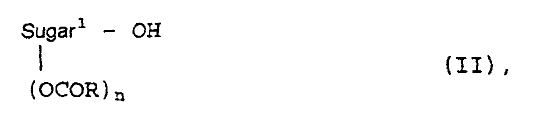 in which sugar ¹, R and n have the indicated meaning,
is reacted with an alkylating reagent of general formula (III)
Nu - L - X - Sg (III),
in which Nu means a nucleofuge, L and X have the above-mentioned meaning, and Sg represents a protective group,
in an organic solvent in the presence of a base and optionally a phase transfer catalyst at a temperature of 0-50°C, then the protective group is cleaved off, and the reaction product that is obtained is optionally converted into a salt.

2. Process according to Claim 1, **characterized in that** a peracylated monosaccharide with 5 to 6 C atoms or its deoxy compound is used as a peracylated 1-OH-sugar of general formula II.

3. Process according to Claim 1 or 2, **characterized in that** peracylated glucose, mannose, galactose, ribose, arabinose, xylose, fucose or rhamnose is used as a peracylated 1-OH-sugar of general formula II.

4. Process according to one of Claims 1 to 3, **characterized in that** as an alkylating reagent of general formula III, one is used in which the nucleofuge means radicals -Cl, -Br, -I, -OTs, -OMs, -OSO₂CF₃, -OSO₂C₄F₉ or -OSO₂C₈F₁₇.

5. Process according to one of Claims 1 to 4, **characterized in that** as an alkylating reagent of general formula III, one is used in which radical L means 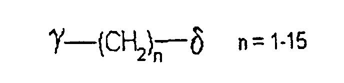 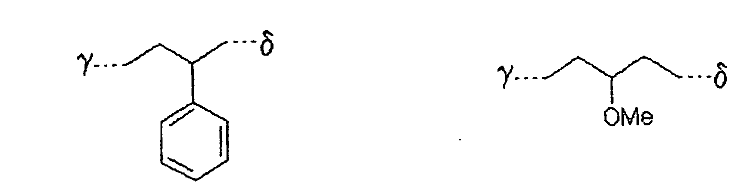 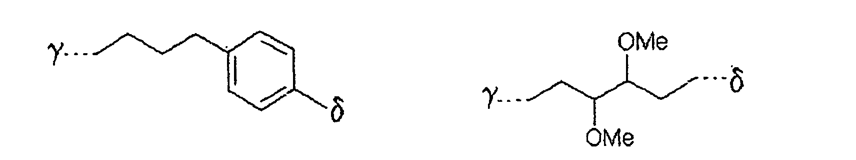 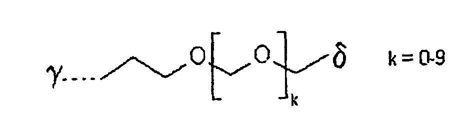 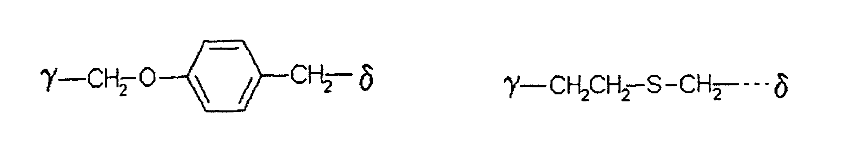 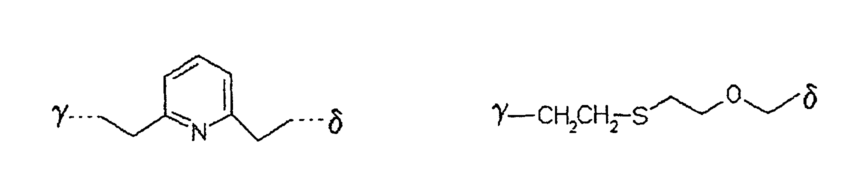 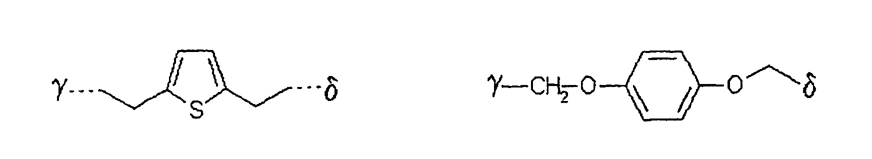 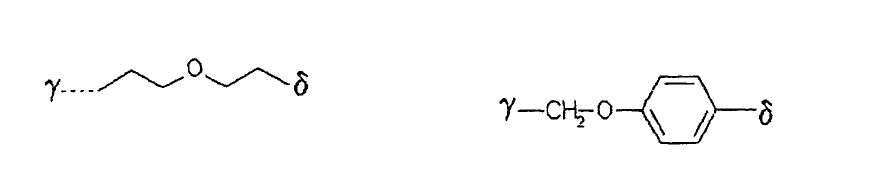 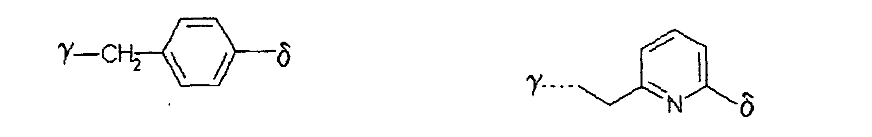 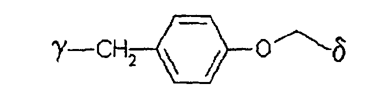 where γ is the interface site to sugar, and δ is the interface site to radical X.

6. Process according to one of Claims 1 to 5, **characterized in that** as an organic solvent, dioxane, 1,2-dimethoxyethane, diethylene glycol dimethyl ether, diethoxymethane, tetrahydrofuran, acetonitrile, formamide, dimethylformamide, dimethylacetamide, benzene, toluene, CF ₃-benzene, hexane, cyclohexane, diethyl ether, dichloromethane, methyl-t-butyl ether (MTB), dimethyl sulphoxide, sulpholane or a mixture thereof is used.

7. Process according to one of Claims 1 to 6, **characterized in that** a quaternary ammonium or phosphonium salt or a crown ether, preferably a quaternary ammonium salt, is used as a phase transfer catalyst.

8. Process according to one of Claims 1 to 7, **characterized in that** potassium carbonate, sodium carbonate, caesium carbonate, 1,8-diazabicyclo[5.4.0]undec-7-ene (DBU), 1,5-diazabicyclo[4.3.0]non-5-ene (DBN), 1,4-diazabicyclo[2.2.2]octane (DABCO), potassium t-butoxide, sodium t-butoxide or a mixture of caesium carbonate and potassium carbonate or sodium carbonate is used as a base.

9. Process according to one of Claims 1 to 7, **characterized in that** the base is added in solid or liquid form.

10. Compounds:
Carboxymethyl-2,3,4,6-tetra-O-acetyl-mannopyranose
Carboxymethyl-2,3,4,6-tetra-O-pivaloyl-mannopyranose
Carboxymethyl-2,3,4, 6-tetra-O-benzoyl-mannopyranose.

## Revendications

1. Procédé pour la préparation de 1-O-glycosides peracylés de formule générale I 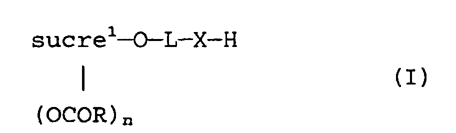 dans laquelle
sucre¹ est un monosaccharide fonctionnalisé en position 1-OH,
R représente le groupe méthyle, éthyle, propyle, isopropyle, tert-butyle, phényle,
n représente 2, 3 ou 4,
X représente -COO- ou -NH-
et
L représente une chaîne carbonée en C ₁-C₃₀ droite, ramifiée, saturée ou insaturée, qui est éventuellement interrompue par 1-10 atomes d'oxygène, 1-3 atomes de soufre, 1-2 groupes phénylène, 1-2 groupes phénylèneoxy, 1-2 groupes phénylènedioxy, un radical thiophène, pyrimidine ou pyridine, et/ou est éventuellement substituée par 1-3 groupes phényle, 1-3 groupes carboxy, 1-5 groupes hydroxy, 1-5 groupes O -alkyle(C₁-C₇), 1-3 groupes amino, 1-3 groupes CF ₃ ou 1-10 atomes de fluor,
ou de leurs sels,
**caractérisé en ce qu'**on fait réagir un 1 -OH-sucre peracylé de formule générale II 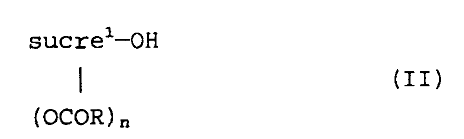 dans laquelle sucre¹, R et n ont les significations indiquées,
avec un réactif d'alkylation de formule générale (III)
Nu-L-X-Sg (III)
dans laquelle Nu représente un nucléofuge, L et X ont les significations données et Sg représente un groupe protecteur
dans un solvant organique en présence d'une base et éventuellement d'un catalyseur de transfert de phase, à une température de 0-50°C, on élimine ensuite le groupe protecteur et le produit de réaction obtenu est éven tuellement converti en un sel.

2. Procédé selon la revendication 1, **caractérisé en ce qu'**on utilise en tant que 1 -OH-sucre peracylé de formule générale II un monosaccharide peracylé ayant 5 ou 6 atomes de carbone ou un composé désoxy de celui-ci.

3. Procédé selon la revendication 1 ou 2, **caractérisé en ce qu'**on utilise en tant que 1 -OH-sucre peracylé de formule générale II du glucose, mannose, galactose, ribose, arabinose, xylose, fucose ou rhamnose, peracylé.

4. Procédé selon l'une quelconque des revendications 1 à 3, **caractérisé en ce qu'**on utilise en tant que réac tif d'alkylation de formule générale III un tel réactif dans lequel le nucléofuge représente les radicaux -Cl, -Br, -I, -OTs, -OMs, -OSO₂CF₃, -OSO₂C₄F₉ ou -OSO₂C₈F₁₇.

5. Procédé selon l'une quelconque des revendications 1 à 4, **caractérisé en ce qu'**on utilise en tant que réactif d'alkylation de formule générale III un tel réactif dans lequel le radical L représente 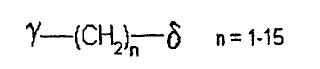 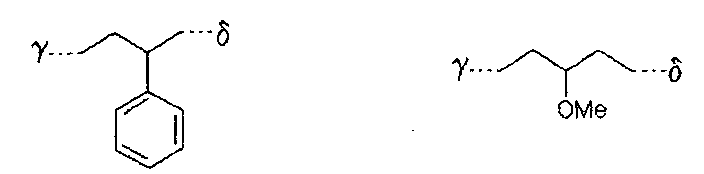 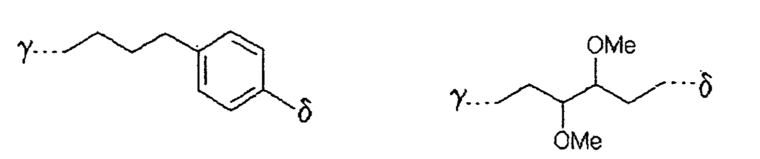 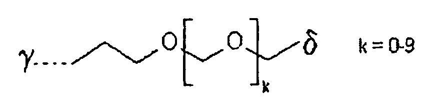 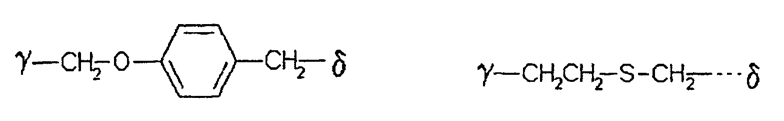 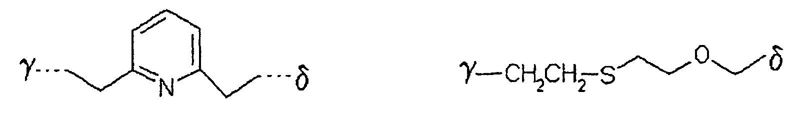 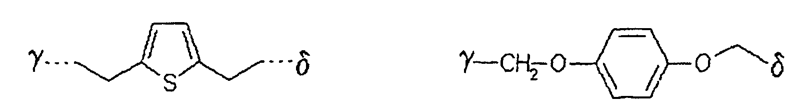 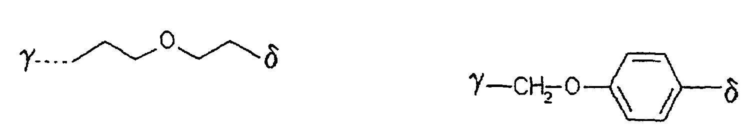 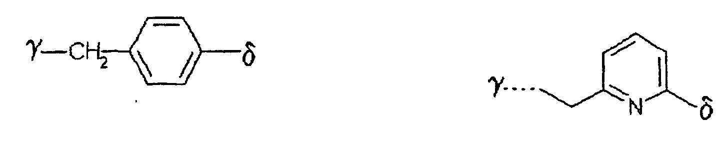 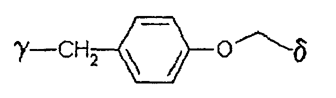 γ représentant le point d'attachement au sucre et δ étant le point d'attachement au radical X.

6. Procédé selon l'une quelconque des revendications 1 à 5, **caractérisé en ce qu'**on utilise en tant que solvant organique le dioxanne, le 1,2-diméthoxy éthane, l'éther diméthylique de diéthylèneglycol, le diéthoxyméthane, le tétrahydrofuranne, l'acétonitrile, le formamide, le diméthylformamide, le diméthylacétamide, le benzène, le toluène, le CF ₃-benzène, l'hexane, le cyclohexane, l'oxyde d'éthyle, le dichlorométhane, l'oxyde de méthyle et de tert-butyle (MTB), le diméthylsulfoxyde, le sulfolane ou des mélanges de ceux-ci.

7. Procédé selon l'une quelconque des revendications 1 à 6, caracté risé en ce qu'on utilise en tant que cata lyseur de transfert de phase un sel d'ammonium ou phos phonium quaternaire ou un éther-couronne, de préférence un sel d'ammonium quaternaire.

8. Procédé selon l'une quelconque des revendications 1 à 7, **caractérisé en ce qu'**on utilise comme base le carbonate de potassium, le carbonate de sodium, le carbonate de césium, le 1,8-diazabicyclo[5.4.0]undéc-7-ène (DBU ), le 1,5-diazabicyclo[4.3.0]non-5-ène (DBN), le 1,4-diazabicyclo[2.2.2]octane (DABCO), le tert-butylate de potassium, le tert-butylate de sodium ou un mélange de carbonate de césium et carbonate de potassium ou carbonate de sodium.

9. Procédé selon l'une quelconque des revendications 1 à 7, **caractérisé en ce que** la base est ajoutée sous forme liquide ou solide.

10. Composés :
carboxyméthyl-2,3,4,6-tétra-O-acétyl-mannopyrannose,
carboxyméthyl-2,3,4,6-tétra-O-pivaloyl-mannopyrannose,
carboxyméthyl-2,3,4,6-tétra-O-benzoyl-mannopyrannose.
